# EUROPÄISCHE PATENTANMELDUNG

(11) **EP 0 866 057 A2**
(43) Veröffentlichungstag der Anmeldung: **23.09.1998**
(21) Anmeldenummer: 98103997.7
(22) Anmeldetag: 06.03.1998
(51) Int. Cl.: C07C 263/10, C07C 263/20, C07C 265/14, C08G 18/70

(54) **Verfahren zur Herstellung von Isocyanaten mit heller Farbe**

(30) Priorität: 19.03.1997 DE 19711447
(71) Anmelder: BASF AKTIENGESELLSCHAFT, 67056 Ludwigshafen (DE)
(72) Erfinder: Kraus, Rupert, Dr., 67165 Waldsee (DE); Reif, Martin, Dr., 67063 Ludwigshafen (DE); Bruchmann, Bernd, Dr., 67251 Freinsheim (DE); Tesch, Helmut, Dr., 67127 Rödersheim-Gronau (DE)

(57) **Zusammenfassung**

Die vorliegende Erfindung betrifft ein Verfahren zur Herstellung von Isocyanaten mit heller Farbe durch Umsetzung der entsprechenden Amine mit Phosgen, dadurch gekennzeichnet, daß die Amine vor der Umsetzung mit dem Phosgen mit festen anorganischen Substanzen, die lewissaure und/oder brönstedtsaure Zentren enthalten, behandelt werden.

## Beschreibung

Die Erfindung betrifft ein Verfahren zur Herstellung von Isocyanaten, insbesondere Polyisocyanaten der Diphenylmethandiisocyanat-Reihe, die eine helle Farbe aufweisen.

Isocyanate sind Rohstoffe für die Herstellung von Polyurethanen. Ihre Herstellung erfolgt zumeist durch Umsetzung der entsprechenden Amine mit Phosgen. Eines der meistverwendeten Isocyanate ist das Diphenylmethandiisocyanat (MDI) sowie seine höheren Homologen (meist als Roh-MDI bezeichnet).

Bedingt durch das Ausgangsamin, das ein Gemisch aus Diphenylmethandiamin und Polyphenylenpolymethylenpolyamin (MDA) darstellt, und durch den MDI-Herstellungsprozeß, der die Umsetzung des MDA mit Phosgen und die nachfolgende Aufarbeitung des Reaktionsproduktes durch Abtrennung der Lösungsmittel und Abdestillation von Monomer-MDI umfaßt, wird ein Roh-MDI erhalten, welches dunkel gefärbt ist und zu gelblich verfärbten Polyurethan-Materialien führt. Da diese Verfärbungen als qualitätsmindernd betrachtet werden, hat es nicht an Versuchen gefehlt, Roh-MDI mit heller Farbe zur Verfügung zu stellen.

So beschreibt US-A-5,364,958 ein Verfahren zur Herstellung von MDI, bei dem das Phosgen nach der Phosgenierung bei niedrigen Temperaturen abgetrennt und das verbleibende Isocyanat zur Zerstörung der Verbindungen, die eine Verfärbung hervorrufen, zumeist als Farbprecursoren bezeichnet, mit HCl-Gas behandelt wird.

EP-A-581 100 beschreibt den Zusatz von chemischen Reduktionsmitteln zum Reaktionsprodukt nach der Phosgenierung, jedoch vor der Abtrennung des Lösungsmittels. Derartige Verfahren bewirken jedoch zumeist nur eine unvollständige Aufhellung des MDI.

Eine weitere Möglichkeit zur Farbaufhellung von MDI besteht im Zusatz von Additiven zum phosgenierten Rohprodukt. So beschreibt US-A-4,465,639 den Zusatz von Wasser, EP-A-538 500 den Zusatz von Carbonsäuren, EP-A-445 602 den Zusatz von Alkanolen und EP-A-467 125 den Zusatz von Polyethern zum Reaktionsprodukt nach der Phosgenierung. Nachteilig ist hierbei, daß es bei der Umsetzung zu unerwünschten Nebenreaktionen kommen kann.

Bekannt ist weiterhin die Behandlung des MDI-Endproduktes nach der Aufarbeitung.

So beschreibt EP-A-133 538 die Reinigung von Isocyanaten durch Extraktion. In EP-A-561 225 und EP-A-676 391 wird die hydrierende Nachbehandlung von MDI zum Zweck der Farbaufhellung beschrieben, wobei in EP-A-676 391 die Verwendung spezieller Hydrierkatalysatoren beschrieben wird. Auch bei diesen Verfahren kommt es nicht zu einer vollständigen Aufhellung der Isocyanate.

Eine weitere Verfahrensmöglichkeit zur Aufhellung von MDI ist die Behandlung des als Rohstoff eingesetzten MDA. In EP-A-546 398 wird vorgeschlagen, das MDA vor der Phosgenierung mit Salzsäure anzusäuern. Der Effekt dieser Maßnahme ist jedoch nur gering. EP-A-446 781 beschreibt ein Verfahren zur Herstellung von hellem MDI durch hydrierende Behandlung des MDA. Auf Grund der drastischen Bedingungen einer solchen Hydrierung kann es jedoch zu störenden Nebenreaktionen kommen.

Weiterhin ist es möglich, die Verfahrensbedingungen bei der MDA-Herstellung zu verändern. Hierbei sind jedoch Nebenreaktionen, die zu einer Veränderung der übrigen Eigenschaften des MDA führen können, nicht auszuschließen.

Aufgabe der Erfindung war es, ein Verfahren zur Herstellung von Isocyanaten, insbesondere Roh-MDI, mit heller Farbe zu entwikkeln, das einfach ist, keine Eingriffe in das laufende Verfahren erfordert und eine wirkungsvolle Farbaufhellung bringt.

Die Aufgabe konnte überraschenderweise gelöst werden durch Behandlung der Amine mit festen anorganischen Substanzen, die lewis-saure und/oder brönstedt-saure Zentren enthalten, und anschließende Phosgenierung der so behandelten Amine.

Gegenstand der Erfindung ist demzufolge ein Verfahren zur Herstellung von Isocyanaten mit heller Farbe durch Umsetzung von Aminen mit Phosgen, dadurch gekennzeichnet, daß die Amine vor der Umsetzung mit Phosgen mit festen anorganischen Substanzen, die lewis-saure und/oder brönstedt-saure Zentren enthalten, behandelt werden.

Als feste anorganische Substanzen, die lewis- und/oder brönstedtsaure Zentren enthalten, werden insbesondere Verbindungen eingesetzt, die eine große innere Oberfläche besitzen und daher gut mit den flüssigen bzw. gelösten Aminen in Kontakt zu bringen sind. Bevorzugt eingesetzt werden natürliche, künstlich modifizierte und künstliche Oxide und/oder Silikate, insbesondere Alumosilikate. Beispiele hierfür sind aktivierte Aluminiumoxide, aktivierte Kieselsäuren und Kieselgele. Bevorzugt sind aktivierte Silikate, insbesondere Aluminiumsilikate, wie z.B. Zeolithe, Bentonite, Kaolinite und Montmorillonite. Ganz besonders bevorzugt sind aktivierte Materialien vom Montmorillonit-Typ.

Die Aktivierung kann thermisch oder chemisch erfolgen. Die chemische Aktivierung ist beispielsweise beschrieben in P. Kumar et. al., "Evolution of Porosity and Surface Acidity in Montmorillonite Clay on Acid Activation", Ind. Eng. Chem. Res., 1995, 1440 - 1448.

Die erfindungsgemäß eingesetzten Substanzen können neben ihrer natürlichen Form besonders bevorzugt auch in künstlich modifizierter Form, z.B. nach chemischer Behandlung mit Säuren oder Metallionen, wie z.B. in P. Laszlo, "Chemical Reactions on Clays, Science 235, 1473 [1987]) beschrieben, eingesetzt werden. Die Partikelgröße und -morphologie der festen anorganischen Substanzen ist für das erfindungsgemäße Verfahren nicht sonderlich kritisch. Es ist jedoch vorteilhaft, eine makroskopische Form dieser Substanzen einzusetzen, die sich nach der Behandlung leicht vom Amin abtrennen läßt. Allgemein gilt: Je feinteiliger das anorganische Material, desto besser ist die farbaufhellende Wirkung und desto schlechter läßt es sich abtrennen und umgekehrt. Die Abtrennung erfolgt zumeist nach den üblichen und bekannten Verfahren zur Entfernung von Feststoffen aus flüssigen Systemen, beispielsweise mittels Filtration, Zentrifugieren oder Dekantieren.

Die festen anorganischen Substanzen können einzeln oder als Gemische eingesetzt werden. Beim Einsatz von Gemischen ist es vorteilhaft, wenn mindestens eine Komponente aus der Gruppe der Montmorillonite stammt.

Prinzipiell wäre es auch möglich, an Stelle der festen anorganischen Substanzen hochpolymere organische Substanzen mit Lewis-und/oder Brönstedt-sauren Zentren zu verwenden. Da jedoch bei den bekannten derartigen Verbindungen störende Wechselwirkungen des Polymergerüstes mit den Aminen und/oder den Lösungsmitteln nicht ausgeschlossen werden können, spielen organische Verbindungen hier keine Rolle.

Als Amine, die der erfindungsgemäßen Behandlung mit den festen anorganischen Substanzen unterworfen werden, sind prinzipiell alle organischen Amine geeignet, die mit Phosgen zu Isocyanaten umgesetzt werden können. Besonders vorteilhaft ist diese Behandlung bei aromatischen Aminen, insbesondere Diphenylmethandiamin (MDA) und dessen höheren Homologen, da insbesondere das Polyphenylenpolymethylenpolyisocyanat (Roh-MDI) zu einer starken Verfärbung neigt.

Die Behandlung der Amine sollte in flüssiger Phase erfolgen, um einen ausreichenden Kontakt zwischen Amin und den festen, anorganischen Substanzen zu gewährleisten. Je nach Konstitution der Amine kann es hierzu vorteilhaft sein, mit Lösungen der Amine zu arbeiten. Als Lösungsmittel können hierbei alle gegenüber Aminen inerten Lösungsmittel verwendet werden, beispielsweise Ester, wie Ethylacetat oder Butylacetat; Ether` wie t-Butyl-methylether, oder Ketone, wie Propanon, Butanon oder Cyclohexanon. Vorzugsweise werden jedoch direkt für den Einsatz im anschließenden Phosgenierungsprozeß geeignete inerte aromatische oder aliphatische Kohlenwasserstoffe bzw. Halogenkohlenwasserstoffe, wie Chlorbenzol, o-Dichlorbenzol, Toluol, Xylole, Alkylbenzole, Alkylnaphthaline, in denen sowohl das Amin als auch das daraus hergestellt Isocyanat löslich sind und die durch die Verfahrensstufen der Amin-Behandlung und der Phosgenierung nicht angegriffen werden, verwendet.

Die Durchführung des erfindungsgemäßen Reinigungsverfahrens kann durch Suspendierung der festen anorganischen Substanzen im Amin bzw. der Aminlösung und anschließende Abtrennung des Feststoffes erfolgen. Es ist jedoch auch möglich, den Feststoff als Festbett anzuordnen und das Amin bzw. die Aminlösung darüberzuleiten.

Die erstgenannte Verfahrensvariante wird zumeist diskontinuierlich durchgeführt. Als Reaktionsgefäße dienen hierbei Mischbehälter, beispielsweise Rührkessel. Nach der Einwirkung des anorganischen Feststoffs auf das Amin wird dieser wie oben beschrieben abgetrennt.

Die Behandlung mittels Festbett wird vorwiegend kontinuierlich durchgeführt, beispielsweise in Kolonnen oder Strömungsrohren.

Die erfindungsgemäße Behandlung des Amins mit anorganischen festen Substanzen erfolgt vorzugsweise bei einem Druck von 0,1 bis 200 bar, vorzugsweise 0,1 bis 10 bar und einer Temperatur von -10°C bis 100°C, vorzugsweise 10 bis 50°C. Die Menge an anorganischer fester Substanz sollte 0,1 bis 30 Gew.-%, vorzugsweise 0,5 bis 15 Gew.-%, bezogen auf die Aminmenge, betragen. Die Zeitdauer der Behandlung liegt bei 1 s bis 5 h, vorzugsweise 1 min bis 1 h.

Die Zeitdauer ist unter anderem abhängig von der Art des eingesetzten Amins und der Wirksamkeit der anorganischen festen Substanz und kann durch Vergleichsversuche ermittelt werden.

Die Abtrennung des Lösungsmittels nach der erfindungsgemäßen Behandlung ist möglich, aber nicht zwingend. Da das Amin zumeist zur Herstellung von Isocyanaten eingesetzt wird, ist es vorteilhaft, die Aminlösung nach der Abtrennung der festen, anorganischen Substanz unmittelbar der Phosgenierung zuzuführen.

Die nach dem erfindungsgemäßen Verfahren behandelten Amine können ohne Probleme zu Isocyanaten mit im Vergleich zu unbehandelten Aminen deutlich hellerer Farbe umgesetzt werden.

Besonders vorteilhaft läßt sich das erfindungsgemäße Verfahren bei der Herstellung von Mischungen aus Diphenylmethandiisocyanat und seinen höheren Homologen, sog. "Roh-MDI", anwenden. Das als Amin eingesetzte Gemisch aus Zweikern- und Mehrkern-MDA, das nach bekannten Verfahren durch Kondensation von Anilin mit Formaldehyd unter Anwesenheit von Salzsäure hergestellt wird, enthält eine große Zahl von Verbindungen, die nach der Umsetzung des MDA zum MDI die dunkle Farbe hervorrufen. Durch die Anwendung des erfindungsgemäßen Verfahrens konnte die Farbe des Roh-MDI deutlich verbessert werden. Das erhaltene Roh-MDI kann zu Polyurethanen mit heller Farbe verarbeitet werden.

Das erfindungsgemäße Verfahren ist einfach durchzuführen und kann leicht in bestehende Anlagen zur Isocyanatherstellung integriert werden. Aufgrund der sehr schonenden Behandlung kommt es zu keinen Nebenreaktionen im Amin und damit zu keinen Beeinträchtigungen der Qualität des Isocyanats. Die festen anorganischen Substanzen können nach der Behandlung praktisch quantitativ vom Amin abgetrennt werden.

Nach der Abtrennung können die festen anorganischen Substanzen nach bekannten Verfahren regeneriert und wiederverwendet oder verworfen werden.

Die Erfindung soll an nachstehenden Beispielen näher erläutert werden:

### Beispiele 1 bis 6

Je 150 g neutralisiertes und getrocknetes MDA aus der sauren Kondensation von Anilin und Formaldehyd wurden in 30 ml Toluol gelöst. Danach wurden 20 g der anorganischen Substanz entsprechend Tabelle 1 zugegeben und die Lösung 10 bis 30 Minuten gerührt. Anschließend wurde über eine Druckfiltrationseinheit bei 3 bar Stickstoff-Überdruck die anorganische Substanz abgetrennt und das klare Filtrat im Vakuum vom Lösungsmittel Toluol befreit. Je 100 g des so behandelten MDA wurden in 1 Liter Monochlorbenzol gelöst und in einem 6-Liter-Rührreaktor mit 200 g Phosgen, gelöst in 1,3 l Monochlorbenzol, bei 50 - 80°C drucklos zum Carbamoylchlorid umgesetzt. Die Temperatur der Reaktionsmischung wurde während 2 h auf 120°C erhöht, wobei die Umsetzung zum Isocyanat erfolgte. Anschließend wurden das Restphosgen und der Hauptanteil Monochlorbenzol abgezogen und danach bei 10 mbar und 120°C das restliche Monochlorbenzol entfernt. Das Reaktionsgemisch wurde danach abgelassen und bei 10 mbar und 180°C 45 min am Rotationsverdampfer thermisch nachbehandelt.

Von den so erhaltenen MDI-Proben wurden die NCO-Gehalte nach DIN 53 185, die EHC- ("easily hydrolizable chlorine") -Werte nach ASTM D 4667-87 und die DHC- ("difficultly hydrolyzable chlorine") nach ASTM D 4663-87 bestimmt.

Außerdem wurde die Jod-Farbzahl nach DIN 6162 bestimmt. Dazu wurden die Proben, verdünnt 1 : 5 in Monochlorbenzol, mit einem Komparatorgerät der Firma Hellige durch Vergleich mit Farbscheiben entsprechend Jod-Farbzahlen untersucht, außerdem wurde eine photometrische Farbzahlbestimmung mittels eines Photometers der Firma Doktor Lange im Jodfarbzahlmodus durchgeführt. Die Ergebnisse sind in Tabelle 1 festgehalten.

**Tabelle 1**

| Bsp. | Zusatz | NCO (%) | EHC (ppm) | DHC (PPM) | IFZ 1:5 *) | FZ 1:5 **) |
|---|---|---|---|---|---|---|
| 1 | Montmorillonit K 10 (20 g) | 32,1 | 237 | 758 | 35 | 19 |
| 2 (V) | - | 32,2 | 316 | 915 | 80 | 41 |
| 3 | Kieselgel 60 (20 g) | 32,0 | 215 | 820 | 50 | 26 |
| 4 | Montmorillonit K 10 (20 g) | 31,9 | 209 | 736 | 30 | 17 |
| 5 | Molekularsieb 10 A (20 g) | 31,8 | 190 | 730 | 35 | 21 |
| 6 | Montmorillonit KSF (20 g) | 31,6 | 182 | 769 | 50 | 25 |

| | | | | | | |
|---|---|---|---|---|---|---|
| V Vergleichsbeispiel | | | | | | |
| IFZ Jodfarbzahl | | | | | | |
| FZ Farbzahl nach photometrischer Bestimmung | | | | | | |
| *) gemessen mittels Komparator | | | | | | |
| **) gemessen mittels Photometer | | | | | | |

## Patentansprüche

1. Verfahren zur Herstellung von Isocyanaten mit heller Farbe durch Umsetzung der entsprechenden Amine mit Phosgen, dadurch gekennzeichnet, daß die Amine vor der Umsetzung mit dem Phosgen mit festen anorganischen Substanzen, die lewissaure und/ oder brönstedtsaure Zentren enthalten, behandelt werden.

2. Verfahren nach Anspruch 1, dadurch gekennzeichnet, daß als feste anorganische Substanzen natürliche, künstlich modifizierte oder künstliche Oxide und/oder Silikate eingesetzt werden.

3. Verfahren nach Anspruch 1 oder 2, dadurch gekennzeichnet, daß als feste anorganische Substanzen Alumosilikate verwendet werden.

4. Verfahren nach einem der Ansprüche 1 bis 3, dadurch gekennzeichnet, daß als feste anorganische Substanzen Montmorillonite verwendet werden.

5. Verfahren nach einem der Ansprüche 1 bis 4, dadurch gekennzeichnet, daß die festen anorganischen Substanzen chemisch und/oder thermisch aktiviert sind.

6. Verfahren nach einem der Ansprüche 1 bis 5, dadurch gekennzeichnet, daß die Aktivierung durch Behandlung der festen anorganischen Substanzen mit Säuren und/oder Metallionen erfolgt.

7. Verfahren nach einem der Ansprüche 1 bis 6, dadurch gekennzeichnet, daß als Amine Diphenylmethandiamin und dessen höhere Homologen eingesetzt werden.

8. Verfahren nach einem der Ansprüche 1 bis 7, dadurch gekennzeichnet, daß die Behandlung bei Drücken zwischen 0,1 und 200 bar und Temperaturen von -10 bis 100°C erfolgt.

9. Verfahren nach einem der Ansprüche 1 bis 8, dadurch gekennzeichnet, daß die Behandlung bei Drücken zwischen 0,1 bis 10 bar und Temperaturen von 10 bis 50°C erfolgt.

10. Verfahren nach einem der Ansprüche 1 bis 9, dadurch gekennzeichnet, daß die Amine zur Behandlung mit den festen anorganischen Substanzen in organischen Lösungsmitteln gelöst werden.

11. Verfahren zur Herstellung von Isocyanaten mit heller Farbe, dadurch gekennzeichnet, daß die entsprechenden Amine Amine, einer Behandlung nach Anspruch 1 bis 10 unterworfen, die feste anorganische Substanz nach der Behandlung abgetrennt wird und nach der Abtrennung der festen anorganischen Substanz die Amine mit Phosgen zum entsprechenden Isocyanat umgesetzt werden.

12. Isocyanate mit heller Farbe, hergestellt durch Umsetzung eines Amins mit Phosgen, dadurch gekennzeichnet, daß das Amin vor der Umsetzung mit Phosgen einer Behandlung nach einem der Ansprüche 1 bis 10 unterworfen wurde.

13. Polyurethane, herstellbar unter Verwendung von Isocyanaten nach Anspruch 12.
